# EUROPEAN PATENT SPECIFICATION

(11) **EP 4 661 925 B1**
(45) Date of publication and mention of the grant of the patent: **21.10.2026**
(21) Application number: 24722354.8
(22) Date of filing: 08.03.2024
(51) Int. Cl.: A61L 29/06, A61L 29/16, A61L 29/08, A61L 29/14

(54) **COATED CATHETER AND METHOD OF COATING**
BESCHICHTETER KATHETER UND BESCHICHTUNGSVERFAHREN
CATHÉTER REVÊTU ET PROCÉDÉ DE REVÊTEMENT

(30) Priority: 10.03.2023 US 202318120330
(43) Date of publication of application: 17.12.2025
(73) Proprietor: Bard Access Systems, Inc., Salt Lake City, UT 84116 (US)
(72) Inventor: HAQUE, Ejaz, Sajedul, Salt Lake City, UT 84103 (US); PAPADOPOULOS, Nicholas, P., Salt Lake City, UT 84109 (US); LAINE, Brendan, Salt Lake City, UT 84103 (US); FREASIER, James, L., Salt Lake City, UT 84109 (US)
(74) Representative: Hoffmann Eitle
(86) International application number: PCT/US2024/019206
(87) International publication number: WO 2024/191858

(56) References cited:
- ROGULSKA MAGDALENA ED - SOHN DAEWON ET AL: "Transparent sulfur-containing thermoplastic polyurethanes with polyether and polycarbonate soft segments", POLYMER BULLETIN, SPRINGER, HEIDELBERG, DE, vol. 75, no. 3, 9 June 2017 (2017-06-09), pages 1211 - 1235, XP036426288, ISSN: 0170-0839, [retrieved on 20170609], DOI: 10.1007/S00289-017-2088-X
- ROGULSKA MAGDALENA ET AL: "New thermoplastic poly(carbonate-urethane)s based on chain extenders with sulfur atoms", CHEMICK ZVESTI - CHEMICAL PAPERS, VEDA, BRATISLAVA, SK, vol. 71, no. 7, 22 December 2016 (2016-12-22), pages 1195 - 1204, XP036255576, ISSN: 0366-6352, [retrieved on 20161222], DOI: 10.1007/S11696-016-0112-5
- ZIANI-CHERIF HOUCINE ET AL: "Visible-light-induced surface graft polymerization via camphorquinone impregnation technique", JOURNAL OF BIOMEDICAL MATERIALS RESEARCH, vol. 59, no. 2, 2 November 2001 (2001-11-02), US, pages 386 - 389, XP093178975, ISSN: 0021-9304, DOI: 10.1002/jbm.1254
- KAMOUN ELBADAWY A ET AL: "Carboxylated camphorquinone as visible-light photoinitiator for biomedical application: Synthesis, characterization, and application", ARABIAN JOURNAL OF CHEMISTRY, ELSEVIER, AMSTERDAM, NL, vol. 9, no. 5, 21 March 2014 (2014-03-21), pages 745 - 754, XP029711203, ISSN: 1878-5352, DOI: 10.1016/J.ARABJC.2014.03.008

## Description

### PRIORITY

This application claims the benefit of priority to U.S. Patent Application No. 18/120,330, filed March 10, 2023.

### BACKGROUND

Rogulska, Polymer Bulletin (2018) 7:1211-1235 relates to "Transparent sulfur-containing thermoplastic polyurethanes with polyether and polycarbonate soft segments". Rogulska et al., Chemical Papers (2017) 71:1195-1204 relates to "New thermoplastic poly(carbonate-urethane)s based on chain extenders with sulfur atoms".

Light-initiated free-radical polymerization for coating medical devices such as catheters makes use of ultraviolet ("UV")-light photoinitiators having relatively high absorptivity in a range of UV light such as UVC (e.g., 100 - 280 nm), UVB (e.g., 280 - 315 nm), UVC (e.g., 315 - 400 nm), or a narrower range thereof. UV-light photoinitiators for such free-radical polymerization includes, for example, tert-butyl peroxybenzoate and benzophenone, which have relatively strong UVC absorption peaks at 232 nm and 254 nm, respectively. While the foregoing UV-light photoinitiators can be effective in generating and transferring free radicals for chain-growth polymerization when the UV-light photoinitiators are irradiated in the range of UV light in which they absorb, the UV light, itself, can degrade materials, thereby limiting substrate choices for coated medical devices. In addition, the UV light poses occupational risks, thereby requiring additional safety measures to limit exposure of workers to the UV light.

Disclosed herein are coated medical devices and methods of coating that make use of visible-light photoinitiators.

### SUMMARY

The scope of protection is defined by the claims.

In a first aspect, the invention relates to a coated catheter according to claims 1-8. The coated catheter includes a tubular substrate and a coating over the tubular substrate. The tubular substrate is of a first polymeric material transparent to electromagnetic radiation in a range of visible light. The first polymeric material is a thermoplastic polyurethane transparent to electromagnetic radiation in the range of visible light from 400 nm to 650 nm. The coating is of a second polymeric material anchored to the tubular substrate by chain ends of the second polymeric material impregnated in the first polymeric material. At least a portion of the chain ends including a spent visible-light photoinitiator.

In some embodiments, the thermoplastic polyurethane includes a hard segment having one or more sulfur-based chain extenders.

In some embodiments, the thermoplastic polyurethane includes a soft segment having a polycarbonate moiety.

In some embodiments, the thermoplastic polyurethane includes a soft segment having a polyether moiety.

In some embodiments, the spent visible-light photoinitiator is spent camphorquinone or a spent analog of camphorquinone.

In some embodiments, at least another portion of the chain ends of the first polymeric material include a spent coinitiator.

In some embodiments, the spent coinitiator is a spent tertiary amine selected from ethyl-4-dimethylaminobenzoate; 4-(dimethylamino) benzonitrile; and 2-(N,N-dimethylamino)ethyl methacrylate.

In some embodiments, the coating of the second polymeric material is over either an abluminal surface or a luminal surface of the tubular substrate.

In some embodiments, the coating of the second polymeric material is over both an abluminal surface and a luminal surface of the tubular substrate.

In some embodiments, the second polymeric material is a polyacrylate salt or ester.

In some embodiments, the second polymeric material is the polyacrylate salt. At least a portion of functionalized sites of the second polymeric material are functionalized with anionic carboxylate and a cationic therapeutic agent as a counterion.

In some embodiments, the therapeutic agent is an antimicrobial agent.

In some embodiments, the therapeutic agent is chlorhexidine.

In some embodiments, at least another portion of the functionalized sites of the second polymeric material are functionalized with anionic carboxylate and a cationic dye as a counterion. The dye provides a visible indication the coating of the second polymeric material is over the tubular substrate.

In some embodiments, the dye doubles as an antifungal agent.

In some embodiments, the dye is ethyl violet.

In some embodiments, the coated catheter further includes a catheter hub and one or more extension legs. The catheter tube includes a proximal end portion disposed in the catheter hub. Each extension leg of the one-or-more extension legs includes a distal end portion disposed in the catheter hub.

In a second aspect the invention relates to a method of manufacturing a coated catheter according to claims 9-15. The method includes obtaining an impregnated tubular substrate of a first polymeric material transparent to electromagnetic radiation in a range of visible light. The first polymeric material is a thermoplastic polyurethane transparent to electromagnetic radiation in the range of visible light from 400 nm to 650 nm. The first polymeric material is impregnated with a visible-light photoinitiator. The method also includes disposing the impregnated tubular substrate in an aqueous solution including a monomer dissolved in the aqueous solution. The method also includes irradiating the impregnated tubular substrate with electromagnetic radiation in the range of visible light to which the first polymeric material is transparent. The photoinitiator initiates a radical polymerization of the monomer upon irradiation of the photoinitiator. The radical polymerization coats the impregnated tubular substrate with a coating of a second polymer material to provide the coated tubular substrate.

In some embodiments, the method further includes disposing a non-impregnated tubular substrate in an organic-solvent solution including the photoinitiator dissolved in the organic-solvent solution. The non-impregnated tubular substrate swells in the organic-solvent solution such that the photoinitiator diffuses into the first polymeric material, thereby impregnating the first polymeric material with the photoinitiator to provide the impregnated tubular substrate in a solvent-swollen form thereof.

In some embodiments, the method further includes disposing the solvent-swollen form of the impregnated tubular substrate in water. Organic solvent diffuses from the first polymeric material into the water, thereby shrinking the solvent-swollen form of the impregnated tubular substrate and trapping the photoinitiator in the first polymeric material.

In some embodiments, the thermoplastic polyurethane includes a hard segment having one or more sulfur-based chain extenders and a soft segment having a polycarbonate moiety.

In some embodiments, the visible-light photoinitiator is camphorquinone or an analog of camphorquinone characterized by its absorption of electromagnetic radiation in the range of visible light from 400 nm to 650 nm.

In some embodiments, the impregnated tubular substrate is further impregnated with a coinitiator, the coinitiator being a tertiary amine selected from ethyl-4-dimethylaminobenzoate; 4-(dimethylamino) benzonitrile; and 2-(N,N-dimethylamino)ethyl methacrylate.

In some embodiments, the coating of the second polymeric material is over either an abluminal surface or a luminal surface of the coated tubular substrate.

In some embodiments, the coating of the second polymeric material is over both an abluminal surface and a luminal surface of the coated tubular substrate.

In some embodiments, the second polymeric material is a polyacrylate salt or ester.

In some embodiments, the method further includes steps or operations for providing additional functionality to the coating of the second polymer material. As such, the method also includes disposing the coated tubular substrate in another aqueous solution including a therapeutic agent. A proton or metal cation is thereby exchanged for a cationic therapeutic agent as a counterion to anionic carboxylate in at least a portion of functionalized sites of the second polymeric material.

In some embodiments, the therapeutic agent is an antimicrobial agent.

In some embodiments, the therapeutic agent is chlorhexidine.

In some embodiments, the other aqueous solution further includes a dye. A proton or metal cation is thereby exchanged for a cationic dye as a counterion to anionic carboxylate in at least another portion of functionalized sites of the second polymeric material.

In some embodiments, the dye doubles as an antifungal agent.

In some embodiments, the dye is ethyl violet.

In some embodiments, the method further includes steps or operations for assembling the coated catheter. As such, the method also includes inserting a proximal end portion of the coated tubular substrate into a catheter hub. The coated tubular substrate corresponds to a catheter tube of the coated catheter. The method also includes inserting a distal end portion of an extension leg into the catheter hub for each extension leg of one or more extension legs of the coated catheter.

These and other features of the concepts provided herein will become more apparent to those of skill in the art in view of the accompanying drawings and following description, which describe particular embodiments of such concepts in greater detail.

### BRIEF DESCRIPTION OF DRAWINGS

FIG. 1 illustrates a coated catheter in accordance with some embodiments.
FIG. 2 illustrates a transverse cross section of a catheter tube of the coated catheter in accordance with some embodiments.
FIG. 3 illustrates a transverse cross section of another catheter tube of the coated catheter in accordance with some embodiments.
FIG. 4 illustrates a transverse cross section of yet another catheter tube of the coated catheter in accordance with some embodiments.
FIG. 5 illustrates a transverse cross section of yet another catheter tube of the coated catheter in accordance with some embodiments.
FIG. 6 provides a schematic of the catheter tube of FIG. 3 with a coating anchored to the catheter tube, an antimicrobial agent ionically bonded to the coating, and a dye ionically bonded to the coating in accordance with some embodiments.
FIG. 7 illustrates a genus of transparent thermoplastic polyurethanes for the catheter tube in accordance with some embodiments.
FIG. 8 illustrates another genus of transparent thermoplastic polyurethanes for the catheter tube in accordance with some embodiments.
FIG. 9 provides a schematic of the catheter tube of FIG. 4 illustrating the coating anchored to the catheter tube by spent visible-light photoinitiator and spent coinitiator in accordance with some embodiments.
FIG. 10 provides a schematic illustrating a method of coating the catheter tube in accordance with some embodiments.
FIG. 11 provides a schematic illustrating a mechanism for anchoring the coating to the catheter tube by the spent visible-light photoinitiator and spent coinitiator in accordance with some embodiments.

### DESCRIPTION

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. In addition, any of the foregoing features or steps can, in turn, further include one or more features or steps unless indicated otherwise. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

"Proximal" is used to indicate a portion, section, piece, element, or the like of a medical device intended to be near or relatively nearer to a clinician when the medical device is used on a patient. For example, a "proximal portion" or "proximal section" of the medical device includes a portion or section of the medical device intended to be near the clinician when the medical device is used on the patient. Likewise, a "proximal length" of the medical device includes a length of the medical device intended to be near the clinician when the medical device is used on the patient. A "proximal end" of the medical device is an end of the medical device intended to be near the clinician when the medical device is used on the patient. The proximal portion, the proximal section, or the proximal length of the medical device need not include the proximal end of the medical device. Indeed, the proximal portion, the proximal section, or the proximal length of the medical device can be short of the proximal end of the medical device. However, the proximal portion, the proximal section, or the proximal length of the medical device can include the proximal end of the medical device. Should context not suggest the proximal portion, the proximal section, or the proximal length of the medical device includes the proximal end of the medical device, or if it is deemed expedient in the following description, "proximal portion," "proximal section," or "proximal length" can be modified to indicate such a portion, section, or length includes an end portion, an end section, or an end length of the medical device for a "proximal end portion," a "proximal end section," or a "proximal end length" of the medical device, respectively.

"Distal" is used to indicate a portion, section, piece, element, or the like of a medical device intended to be near, relatively nearer, or even in a patient when the medical device is used on the patient. For example, a "distal portion" or "distal section" of the medical device includes a portion or section of the medical device intended to be near, relatively nearer, or even in the patient when the medical device is used on the patient. Likewise, a "distal length" of the medical device includes a length of the medical device intended to be near, relatively nearer, or even in the patient when the medical device is used on the patient. A "distal end" of the medical device is an end of the medical device intended to be near, relatively nearer, or even in the patient when the medical device is used on the patient. The distal portion, the distal section, or the distal length of the medical device need not include the distal end of the medical device. Indeed, the distal portion, the distal section, or the distal length of the medical device can be short of the distal end of the medical device. However, the distal portion, the distal section, or the distal length of the medical device can include the distal end of the medical device. Should context not suggest the distal portion, the distal section, or the distal length of the medical device includes the distal end of the medical device, or if it is deemed expedient in the following description, "distal portion," "distal section," or "distal length" can be modified to indicate such a portion, section, or length includes an end portion, an end section, or an end length of the medical device for a "distal end portion," a "distal end section," or a "distal end length" of the medical device, respectively.

Unless defined otherwise, all technical and scientific terms used herein have the same meaning as commonly understood by those of ordinary skill in the art.

Again, light-initiated free-radical polymerization for coating medical devices such as catheters makes use of UV-light photoinitiators having relatively high absorptivity in a range of UV light such as UVC (e.g., 100 - 280 nm), UVB (e.g., 280 - 315 nm), UVC (e.g., 315 - 400 nm), or a narrower range thereof. UV-light photoinitiators for such free-radical polymerization includes, for example, *tert-butyl* peroxybenzoate and benzophenone, which have relatively strong UVC absorption peaks at 232 nm and 254 nm, respectively. While the foregoing UV-light photoinitiators can be effective in generating and transferring free radicals for chain-growth polymerization when the UV-light photoinitiators are irradiated in the range of UV light in which they absorb, the UV light, itself, can degrade materials, thereby limiting substrate choices for coated medical devices. In addition, the UV light poses occupational risks, thereby requiring additional safety measures to limit exposure of workers to the UV light.

Disclosed herein are coated medical devices and methods of coating that make use of visible-light photoinitiators, thereby at least expanding substrate choices for the coated medical devices and reducing the occupational risks associated with using higher-energy UV light. Coated medical devices include, without limitation, coated catheters such as peripherally inserted central catheters ('PICCs"). For example, a coated catheter can include a catheter tube of a tubular substrate and a coating thereover. The tubular substrate can be of a first polymeric material transparent to electromagnetic radiation in a range of visible light. The coating can be of a second polymeric material anchored to the tubular substrate by chain ends of the second polymeric material impregnated in the first polymeric material by way of a spent visible-light photoinitiator. Methods of coating can include, without limitation, methods of coating medical devices such as the foregoing coated catheter. For example, a method of coating can include irradiating a tubular substrate impregnated with a visible-light photoinitiator with the foregoing electromagnetic radiation while the tubular substrate is disposed in an aqueous solution of a monomer, thereby initiating a radical polymerization of the monomer and coating the tubular substrate with the coating of the second polymer material.

### Medical devices

Coated medical devices encompass any medical device having a coating like that described herein on an intracorporeal portion of the medical device or an entirety thereof, wherein the intracorporeal portion or entirety of the medical device is configured to reside within a patient for a period of time such as during treatment of the patient. For example, the coated medical devices can include coated catheters, which, in turn, can include coated peripheral (intra)venous catheters ("PIVCs") and coated central venous catheters ("CVCs"). Coated CVCs, in turn, can include coated ports, coated percutaneous CVCs configured for insertion through skin into a jugular or subclavian vein, coated rapidly insertable central catheters ("RICCs"), coated PICCs configured for insertion through skin into a vein of an arm, and subcutaneous or tunneled CVCs having a coating like that described herein.

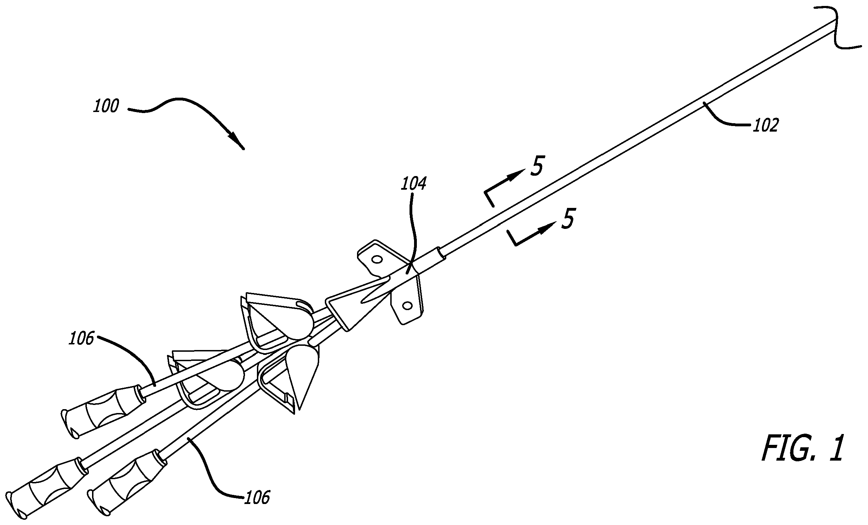

FIG. 1 illustrates a coated catheter 100 in accordance with some embodiments. More specifically, FIG. 1 illustrates a coated PICC in accordance with some embodiments. However, it should be understood that description for the coated catheter 100 is not limited to PICCs but extends to the other coated catheters set forth above as well as other coated medical devices. For example, description of the coating 110 on the catheter tube 102 set forth below extends to a coating on a catheter tube of another coated catheter such as any other coated catheter set forth above. And being that the catheter tube 102 of the coated catheter 100 is the intracorporeal portion thereof, the description of the coating 110 on the catheter tube 102 set forth below further extends to a coating on the intracorporeal portion of another coated medical device.

The coated catheter 100 includes a catheter tube 102, a catheter hub 104, and one or more extension legs 106 operably connected in the foregoing order. Indeed, the catheter tube 102 includes a proximal end portion disposed in the catheter hub 104, and each extension leg of the one-or-more extension legs 106 includes a distal end portion disposed in the catheter hub 104.

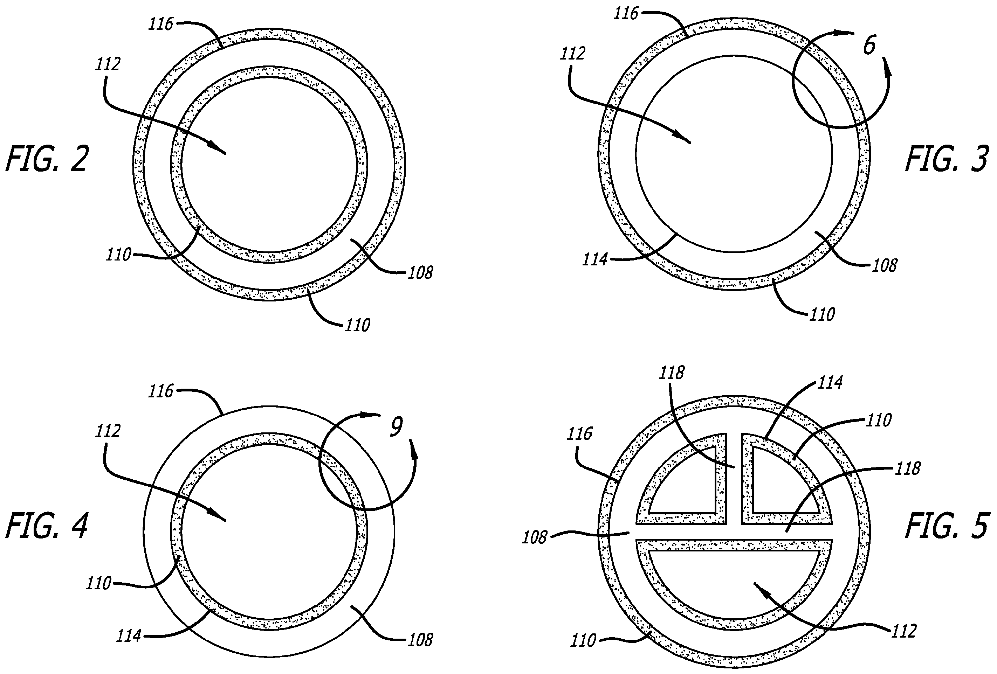

FIGS. 2-5 illustrate transverse cross sections of the catheter tube 102 of the coated catheter 100 in accordance with some embodiments. As shown, the catheter tube 102 includes a tubular substrate 108 and a coating 110 over the tubular substrate 108, which tubular substrate 108 corresponds to the coated tubular substrate 132 set forth below in at least some embodiments. The tubular substrate 108 includes at least one lumen 112 therethrough, the foregoing lumen 112 thereby defining at least one luminal surface 114 of the tubular substrate 108 as well as an abluminal surface 116 of the tubular substrate 108. Additional lumens of the tubular substrate 108 are defined by one or more longitudinal septa 118 dividing the foregoing lumen 112 as shown in FIG. 5. Each additional lumen of the additional lumens includes a corresponding luminal surface. The coating 110 can be over any one or more surfaces selected from the abluminal surface 116 and any luminal surface of the tubular substrate 108. In an example, the coating 110 can be over the abluminal surface 116 and each luminal surface of the tubular substrate 108 as shown in FIGS. 2 and 5. In another example, the coating 110 can be over the abluminal surface 116 of the tubular substrate 108 as shown in FIG. 3. In yet another example, the coating 110 can be over the luminal surface 114 of the tubular substrate 108 as shown in FIG. 4.

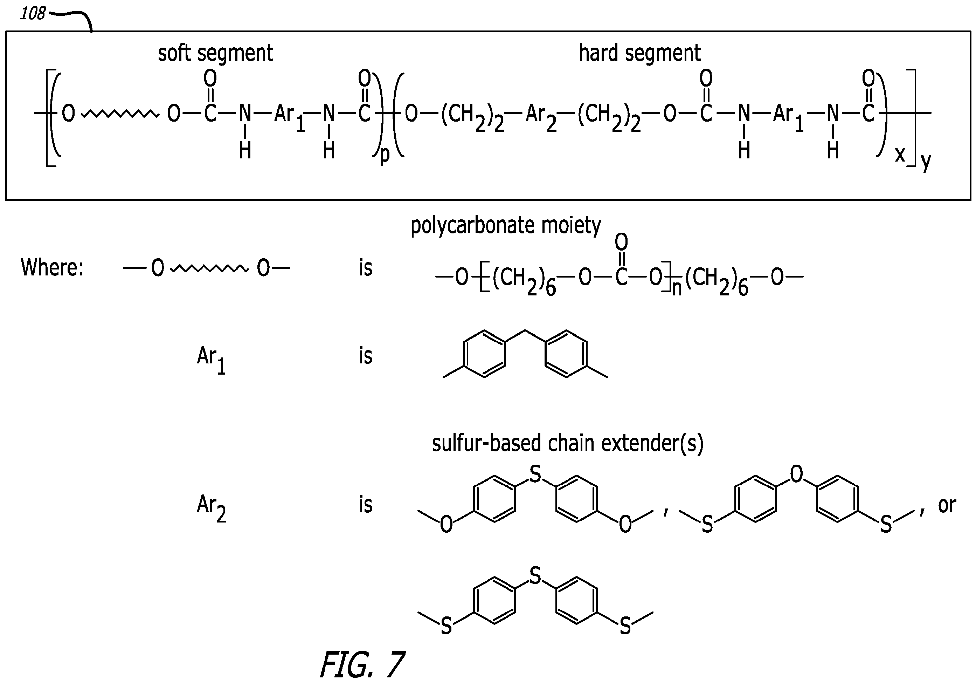

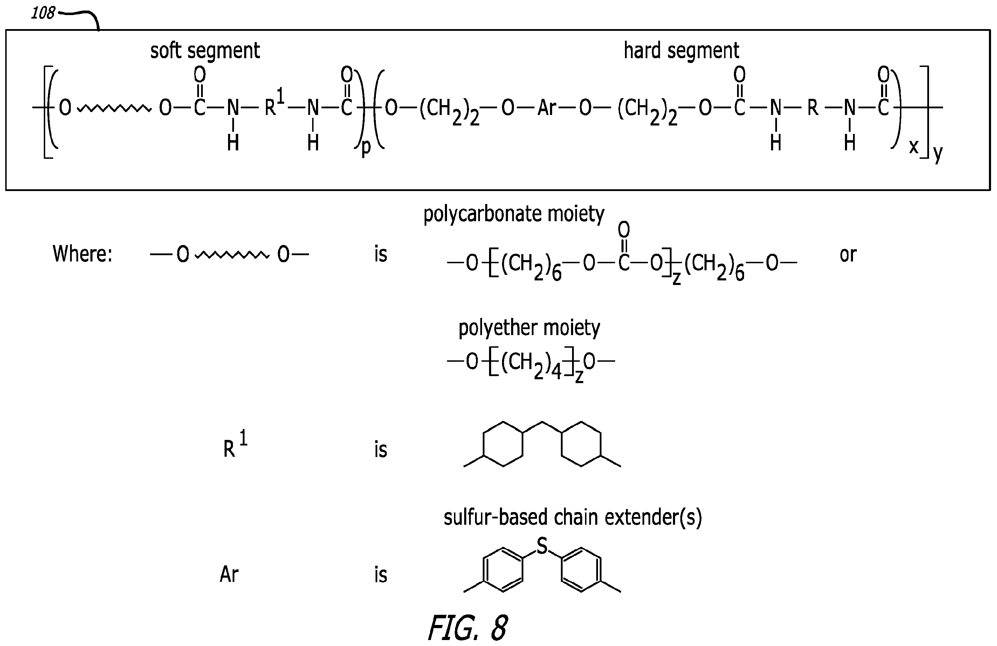

The tubular substrate 108 is of a first polymeric material transparent to electromagnetic radiation in a range of visible light. For example, the first polymeric material can be a thermoplastic polyurethane transparent to electromagnetic radiation in the range of visible light from 400 nm to 650 nm, including 400 nm to 550 nm, such as 450 nm to 500 nm, for example, 460 nm to 470 nm. As shown in FIGS. 7 and 8, such a thermoplastic polyurethane can include a hard segment having one or more sulfur-based chain extenders and a soft segment having a polycarbonate or a polyether moiety. Some of the thermoplastic polyurethanes encompassed by FIGS. 7 and 8 can be found in the following references along with their preparation: 1) Rogulska et al. Chem. Pap. 2017, 71, 1195-1204; and Rogulska et al. Polym. Bull. 2018, 75, 1211-1235. However, it should be understood the first polymeric material is not limited to the thermoplastic polyurethanes of FIGS. 7 and 8. Notably, the tubular substrate 108 of the first polymeric material can include one or more dyes or pigments integrated therein so long as the tubular substrate 108 remains transparent to electromagnetic radiation in the foregoing range(s) of visible light.

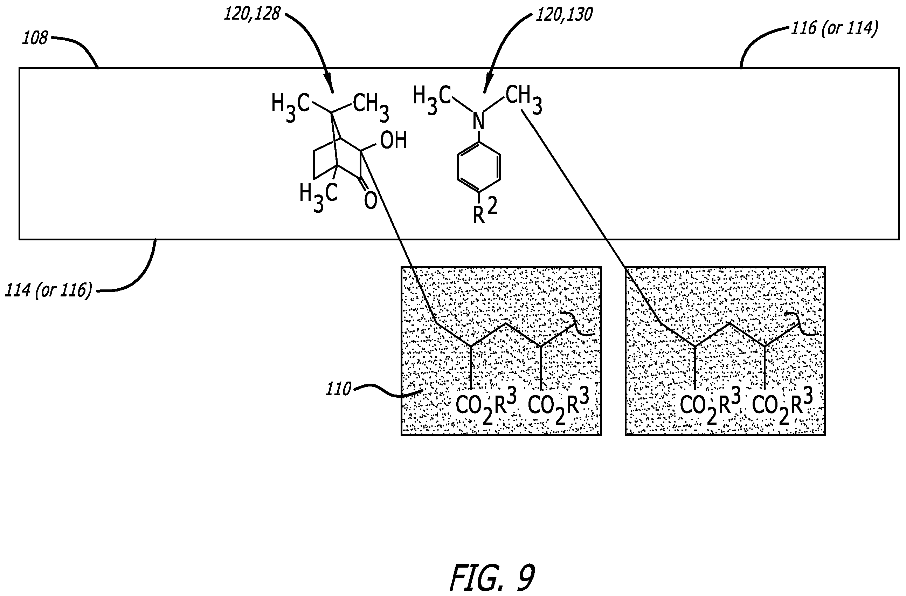

The coating 110 is of a second polymeric material anchored to the tubular substrate 108 by chain ends 120 of the second polymeric material impregnated in the first polymeric material, which advantageously make the coating 110 of the second polymeric material resistant to delamination. The second polymeric material can be a polyacrylic acid (-R³ is -H in FIG. 9), polyacrylate salt, polyacrylate ester, or combination thereof, wherein functionalized sites 122 of the second polymeric material are functionalized with a combination of functional groups selected from carboxy, carboxylate salt, and carboxylate ester in the foregoing combination. The acrylic acid or acrylate residue of the second polymeric material is either unsubstituted or substituted with an alkyl, cycloalkyl, aryl, or heteroaryl group. When present, the carboxylate salt includes one or more counterions selected from a metal cation, cationic therapeutic agent 124, and cationic dye 126. Further, when present, the carboxylate ester includes an organyl group (e.g., -R³ in FIG. 9) selected from an alkyl, cycloalkyl, aryl, or heteroaryl group.

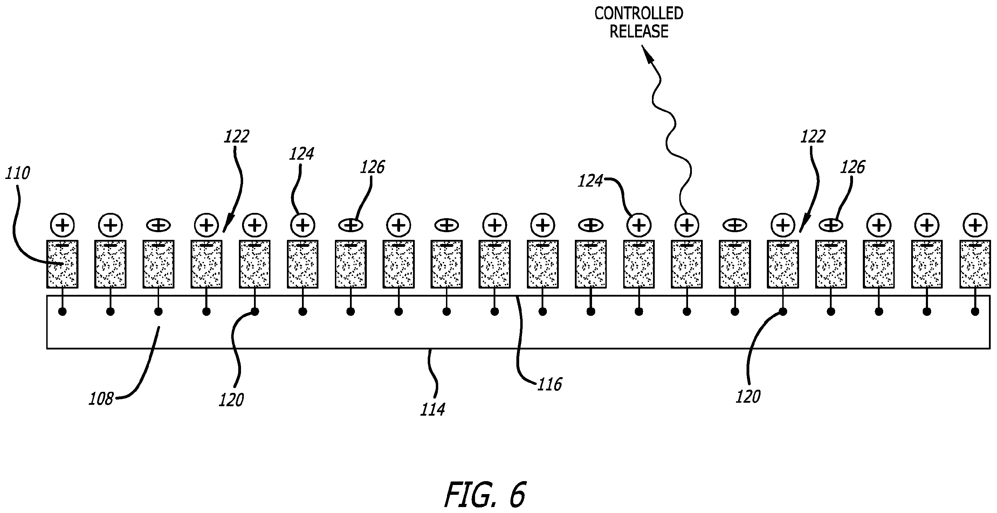

Advantageously, when the second polymeric material includes the polyacrylate salt, at least a portion of the functionalized sites 122 of the second polymeric material functionalized with anionic carboxylate can include one of the cationic therapeutic agent 124 or the cationic dye 126 as the counterion, for example, the cationic therapeutic agent 124. Further, at least another portion of the functionalized sites 122 of the second polymeric material can include the other one of the cationic therapeutic agent 124 or the cationic dye 126 as the counterion, for example, the cationic dye 126. Such an example is shown in the schematic of FIG. 6, wherein at least a portion of the functionalized sites 122 of the second polymeric material include the cationic therapeutic agent 124 as the counterion and at least another portion of the functionalized sites 122 of the second polymeric material include the cationic dye 126 as the counterion, the cationic therapeutic agent 124 and the cationic dye 126 thereby ionically bonded to the coating 110 or the second polymeric material thereof. The cationic therapeutic agent 124 can include, but is not limited to, an antimicrobial agent such as an antimicrobial small molecule, for example, chlorhexidine, or an antimicrobial peptides for example, dalbavancin, daptomycin, oritavancin, teicoplanin, telavancin, or vancomycin, which antimicrobial agent can be slowly or controllably released from the coating 110 or the second polymeric material thereof to prevent bacterial colonization, thereby addressing catheter-related bloodstream infections ("CRBSIs"). The cationic dye 126 can include, but is not limited to, a triarylmethane dye, for example, a methyl violet dye (e.g., methyl violet 10B) or ethyl violet. The cationic dye 126 functions to provide a visible indication the coating 110 of the second polymeric material is present over the tubular substrate 108, but the cationic dye 126 can also function as a therapeutic agent in some embodiments. For example, each of cationic dye 126 of methyl violet 10B and ethyl violet can double as an antimicrobial or antifungal agent, which can enhance any antimicrobial activity of the cationic therapeutic agent 124 with additional antimicrobial activity or complement any antimicrobial activity of the cationic therapeutic agent 124 with antifungal activity when both the cationic therapeutic agent 124 and the cationic dye 126 are ionically bonded to the coating 110 or the second polymeric material thereof.

As to the second polymeric material being anchored to the tubular substrate 108 by the chain ends 120 of the second polymeric material impregnated in the first polymeric material, it should be understood the chain ends 120 are primarily initiation ends of polymer chains of the second polymeric material as opposed to termination ends of the polymer chains. That is, the chain ends 120 of the second polymeric material impregnated in the first polymeric material are those from which the polymer chains of the second polymeric material propagated. Being that the tubular substrate 108 is impregnated with the photoinitiator 136 prior to initiating the radical polymerization set forth in the method below, at least a portion of the chain ends 120 of the second polymeric material impregnated in the first polymeric material include a spent visible-light photoinitiator 128 from which the polymer chains of the second polymeric material propagated. Such a spent visible-light photoinitiator 128 can include spent camphorquinone as shown in FIG. 9 or a spent analog of camphorquinone such as spent carboxylated camphorquinone. Further, being that the tubular substrate 108 can be impregnated with the coinitiator 142 prior to initiating the radical polymerization set forth in the method below, at least another portion of the chain ends 120 of the second polymeric material impregnated in the first polymeric material can include a spent coinitiator 130 from which the polymer chains of the second polymeric material propagated. Such a spent coinitiator 130 can include a spent tertiary amine selected from spent ethyl-4-dimethylaminobenzoate, 4-(dimethylamino) benzonitrile, and 2-(N,N-dimethylamino)ethyl methacrylate. Indeed, FIG. 9 shows spent ethyl-4-dimethylaminobenzoate when -R² is -CO₂CH₂CH₃ and spent 4-(dimethylamino) benzonitrile when -R² is -CN. Even further, should the tubular substrate 108 be impregnated with a polymerization accelerator prior to initiating the radical polymerization set forth in the method below, at least another portion of the chain ends 120 of the second polymeric material impregnated in the first polymeric material can include a spent polymerization accelerator. Such a spent polymerization accelerator can include spent diphenyliodonium chloride, which can be indicated by the chain ends 120 of the second polymeric material including phenyl groups.

### Methods

Methods include methods of manufacturing coated medical devices, which methods, in turn, include methods of coating medical devices or pieces thereof in the manufacturing of the coated medical devices. For example, the methods of manufacturing coated medical devices include a method of manufacturing a coated catheter such as the coated catheter 100, which method, in turn, includes a method of coating a catheter or a piece thereof in the manufacturing of the coated catheter 100. However, like that set forth above it should be understood that description for the method of manufacturing the coated catheter 100, again, a PICC, is not limited to manufacturing PICCs but extends to manufacturing the other coated catheters set forth above as well as manufacturing the other coated medical devices. For example, description of coating the catheter tube 102 set forth below extends to coating a catheter tube of another coated catheter such as any other coated catheter set forth above. And being that the catheter tube 102 of the coated catheter 100 is the intracorporeal portion thereof, the description of coating the catheter tube 102 set forth below further extends to coating the intracorporeal portion of another coated medical device.

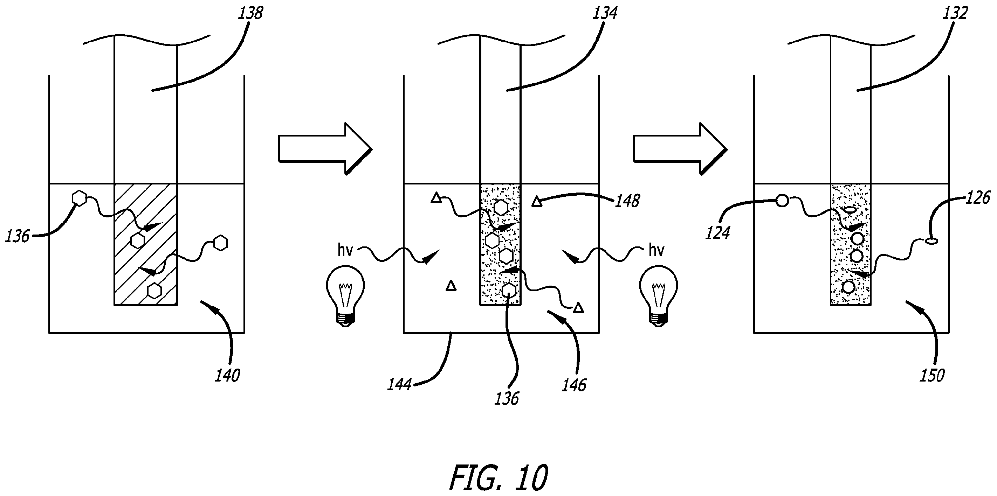

The method of manufacturing the coated catheter 100 includes, like that set forth above, a method of coating a piece of the coated catheter 100 such as the catheter tube 102, which is followed by a method of assembling the coated catheter 100 with the foregoing catheter tube 102. As set forth below, such a method of manufacturing the coated catheter 100 includes various steps or operations beginning with the steps or operations for providing a coated tubular substrate 132 as shown in FIG. 10, which steps or operations can include an impregnating operation, a coating operation, and a functionalizing operation. Such a method of manufacturing the coated catheter 100 includes various steps or operations continuing with the steps or operations for assembling the coated catheter 100 with the foregoing coated tubular substrate 132 as the catheter tube 102, optionally after cutting the coated tubular substrate 132 to size and tipping it with a catheter tip.

As to the impregnating operation, it can include obtaining an impregnated tubular substrate 134 of the first polymeric material impregnated with at least a visible-light photoinitiator 136. Obtaining the impregnated tubular substrate 134 includes disposing a non-impregnated tubular substrate 138 of the first polymeric material in an organic-solvent solution 140 including the photoinitiator 136 dissolved in the organic-solvent solution 140 by a relatively polar organic solvent or mixture of organic solvents, including a mixture of protic or aprotic organic solvents, such as a mixture of protic and aprotic organic solvents, for example, a mixture of 2-propanol and 2-butanone. Upon disposing the non-impregnated tubular substrate 138 in the organic-solvent solution 140, the non-impregnated tubular substrate 138 swells in the organic-solvent solution 140, thereby allowing the photoinitiator 136 to diffuse into the first polymeric material, which, in turn, impregnates the first polymeric material with the photoinitiator 136 to provide the impregnated tubular substrate 134 in a solvent-swollen form thereof. Notably, any lumen of the non-impregnated tubular substrate 138 can be stoppered or otherwise closed off before disposing the non-impregnated tubular substrate 138 in the organic-solvent solution 140 to prevent the photoinitiator 136 from entering the lumen and diffusing into the first polymeric material of the corresponding luminal surface, thereby providing control over which luminal surfaces of the coated tubular substrate 132 are coated. Subsequently disposing the solvent-swollen form of the impregnated tubular substrate 134 in water allows the organic solvent to diffuse from the first polymeric material into the water, thereby shrinking the solvent-swollen form of the impregnated tubular substrate 134 and trapping the photoinitiator 136 in the first polymeric material to provide the impregnated tubular substrate 134 of the first polymeric material impregnated with at least the photoinitiator 136.

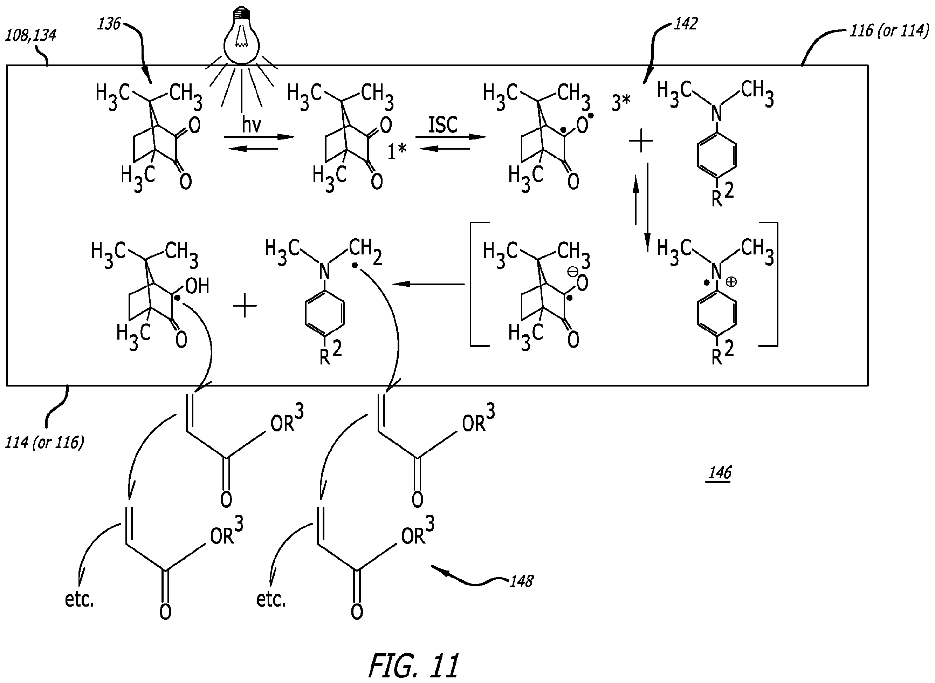

The photoinitiator 136 can be characterized by its absorption of electromagnetic radiation in the range of visible light from 400 nm to 650 nm, including 400 nm to 550 nm, such as 450 nm to 500 nm, for example, 460 nm to 470 nm. Such a photoinitiator 136 can include, but is not limited to, camphorquinone as shown in FIG. 11 or an analog thereof such as carboxylated camphorquinone. *(See* Kamoun et al. Arab. J. Chem. 2016, 9(5), 745-754. Because initiation of radical polymerization with visible-light photoinitiators such as camphorquinone can benefit from coinitiators, the organic-solvent solution 140 can further include a coinitiator 142 dissolved in the organic-solvent solution 140 along with the photoinitiator 136, which coinitiator 142 likewise impregnates the first polymeric material as set forth above when the coinitiator 142 is present in the organic-solvent solution 140. Such a coinitiator 142 can include, but is not limited to, a tertiary amine selected from ethyl-4-dimethylaminobenzoate, 4-(dimethylamino) benzonitrile, and 2-(N,N-dimethylamino)ethyl methacrylate. Indeed, FIG. 11 shows ethyl-4-dimethylaminobenzoate when -R² is - CO₂CH₂CH₃ and 4-(dimethylamino) benzonitrile when -R² is -CN. Lastly, because radical polymerization with visible-light photoinitiators such as camphorquinone can be accelerated with polymerization accelerators, the organic-solvent solution 140 can further include a polymerization accelerator dissolved in the organic-solvent solution 140 along with the photoinitiator 136, which polymerization accelerator likewise impregnates the first polymeric material as set forth above when the polymerization accelerator is present in the organic-solvent solution 140. Such a polymerization accelerator can include, but is not limited to, diphenyliodonium chloride.

As to the coating operation, it can include disposing the impregnated tubular substrate 134 in a suitable reactor 144 (e.g., a quartz reactor) including an aqueous solution 146 having a monomer 148 dissolved in the aqueous solution 146 and irradiating the impregnated tubular substrate 134 with one or more lamps or light-emitting diodes ("LEDs"), thereby providing electromagnetic radiation in the range of visible light to which the first polymeric material is transparent and the photoinitiator 136 absorbs. The photoinitiator 136, which remains trapped in the impregnated tubular substrate 134 in accordance with the swell-preventing aqueous solution 146 of the monomer 148, initiates a radical polymerization of the monomer 148 from the impregnated tubular substrate 134 or wall thereof including the photoinitiator 136 upon irradiation of the photoinitiator 136. As shown in FIG. 11 by way of camphorquinone as the photoinitiator 136 and ethyl-4-dimethylaminobenzoate (when -R² is - CO₂CH₂CH₃) or 4-(dimethylamino) benzonitrile (when -R² is -CN) as the coinitiator 142, upon absorption of the electromagnetic radiation in the range of visible light to which the photoinitiator 136 absorbs (e.g., 468 nm for camphorquinone), an excited singlet state of the photoinitiator 136 is formed, and, through intersystem crossing, a triplet state of the photoinitiator 136 is subsequently formed, thereby providing a diradical of the photoinitiator 136. Electron transfer between the diradical of the photoinitiator 136 and the coinitiator 142 results in an exciplex between a radical anion of the photoinitiator 136 and a radical cation of the coinitiator 142. Further, by way of proton transfer within the exciplex, a ketyl radical of the photoinitiator 136 is formed along with an alkyl radical of the coinitiator 142, both of which are capable of propagating their radicals in the radical polymerization of the monomer 148. Indeed, as shown in FIG. 11, each radical of the ketyl and alkyl radicals can react with the monomer 148 in the aqueous solution 146 in which the impregnated tubular substrate 134 is disposed to coat the impregnated tubular substrate 134 with the second polymer material, thereby providing the coated tubular substrate 132 with the second polymer material anchored to the coated tubular substrate 132.

As set forth above, the second polymeric material anchored to the coated tubular substrate 132 can be a polyacrylic acid, polyacrylate salt, polyacrylate ester, or combination thereof. As such, the monomer 148 dissolved in the aqueous solution 146 into which the impregnated tubular substrate 134 is disposed can be an acrylic acid (-R³ is -H in FIG. 11) or acrylate ester. The acrylic acid or acrylate ester can be either unsubstituted or substituted with an alkyl, cycloalkyl, aryl, or heteroaryl group. Further, when the acrylate ester is present, the organyl group (e.g., -R³ in FIG. 11) of the acrylate ester is selected from an alkyl, cycloalkyl, aryl, or heteroaryl group.

As to the functionalizing operation, it can include modifying the functionalized sites 122 of the second polymer material such as those functionalized with anionic carboxylate to include the one-or-more counterions set forth above, the one-or-more counterions selected from the metal cation, cationic therapeutic agent 124, and cationic dye 126. Accordingly, the functionalizing operation can include disposing the coated tubular substrate 132 in another aqueous solution 150 including the metal cation, cationic therapeutic agent 124, cationic dye 126, or a combination thereof. In an example, the functionalizing operation can include disposing the coated tubular substrate 132 in the other aqueous solution 150 including the cationic therapeutic agent 124, thereby exchanging a proton or metal cation for the cationic therapeutic agent 124 as the counterion to anionic carboxylate in at least a portion of the functionalized sites 122 of the second polymeric material. In another example, the functionalizing operation can include disposing the coated tubular substrate 132 in the other aqueous solution 150 including the cationic dye 126, thereby exchanging a proton or metal cation for the cationic dye 126 as the counterion to anionic carboxylate in at least a portion of the functionalized sites 122 of the second polymeric material. In yet another example, the functionalizing operation can include disposing the coated tubular substrate 132 in the other aqueous solution 150 including both the cationic therapeutic agent 124 and the cationic dye 126, thereby exchanging a proton or metal cation for the cationic therapeutic agent 124 as the counterion to anionic carboxylate in at least a portion of the functionalized sites 122 of the second polymeric material as well as exchanging a proton or metal cation for the cationic dye 126 as the counterion to anionic carboxylate in another portion of the functionalized sites 122 of the second polymeric material.

As to an assembling operation, it can include inserting a proximal end portion of the coated tubular substrate 132 into the catheter hub 104, optionally after cutting the coated tubular substrate 132 to size and tipping it, as set forth above. Such a coated tubular substrate 132 thereby corresponds to the catheter tube 102 of the coated catheter 100. Further, the assembling operation can include inserting a distal end portion of an extension leg into the catheter hub 104 for each extension leg of the one-or-more extension legs 106 of the coated catheter 100.

## Claims

1. A coated catheter (100), comprising:
a catheter tube (102) including:
a tubular substrate (108) of a first polymeric material transparent to electromagnetic radiation in a range of visible light, wherein the first polymeric material is a thermoplastic polyurethane transparent to electromagnetic radiation in the range of visible light from 400 nm to 650 nm; and
a coating (110) of a second polymeric material over the tubular substrate, the coating anchored to the tubular substrate by chain ends of the second polymeric material impregnated in the first polymeric material, at least a portion of the chain ends including a spent visible-light photoinitiator.

2. The coated catheter of claim 1, wherein the thermoplastic polyurethane includes a hard segment having one or more sulfur-based chain extenders; and/or
wherein the thermoplastic polyurethane includes a soft segment having a polycarbonate moiety; or
wherein the thermoplastic polyurethane includes a soft segment having a polyether moiety.

3. The coated catheter of any of the preceding claims, wherein the spent visible-light photoinitiator is spent camphorquinone or a spent analog of camphorquinone.

4. The coated catheter of any of the preceding claims, wherein at least another portion of the chain ends of the first polymeric material include a spent coinitiator;
optionally, wherein the spent coinitiator is a spent tertiary amine selected from ethyl-4-dimethylaminobenzoate; 4-(dimethylamino) benzonitrile; and 2-(N,N-dimethylamino)ethyl methacrylate.

5. The coated catheter of any of the preceding claims, wherein the coating (110) of the second polymeric material is over either an abluminal surface (116) or a luminal surface of the tubular substrate (108); or
wherein the coating (110) of the second polymeric material is over both an abluminal (116) surface and a luminal surface of the tubular substrate (108).

6. The coated catheter of any of the preceding claims, wherein the second polymeric material is a polyacrylate salt or ester;
optionally, wherein the second polymeric material is the polyacrylate salt, at least a portion of functionalized sites of the second polymeric material being functionalized with anionic carboxylate and a cationic therapeutic agent as a counterion;
optionally, wherein the therapeutic agent is an antimicrobial agent;
optionally, wherein the therapeutic agent is chlorhexidine.

7. The coated catheter of claim 6, wherein at least another portion of the functionalized sites of the second polymeric material are functionalized with anionic carboxylate and a cationic dye as a counterion, thereby providing a visible indication the coating (110) of the second polymeric material is over the tubular substrate (108);
optionally, wherein the dye doubles as an antifungal agent;
optionally, wherein the dye is ethyl violet.

8. The coated catheter of any of the preceding claims, further comprising:
a catheter hub (104), the catheter tube (102) including a proximal end portion disposed in the catheter hub (104); and
one or more extension legs (106), each extension leg of the one-or-more extension legs (106) including a distal end portion disposed in the catheter hub (104).

9. A method of manufacturing a coated catheter (100), comprising:
obtaining an impregnated tubular substrate (134) of a first polymeric material transparent to electromagnetic radiation in a range of visible light, wherein the first polymeric material is a thermoplastic polyurethane transparent to electromagnetic radiation in the range of visible light from 400 nm to 650 nm, the first polymeric material impregnated with a visible-light photoinitiator;
disposing the impregnated tubular substrate (134) in an aqueous solution including a monomer dissolved in the aqueous solution; and
irradiating the impregnated tubular substrate (134) with electromagnetic radiation in the range of visible light to which the first polymeric material is transparent, the photoinitiator initiating a radical polymerization of the monomer upon irradiation of the photoinitiator, thereby coating the impregnated tubular substrate (134) with a coating (110) of a second polymer material to provide a coated tubular substrate (132).

10. The method of claim 9, further comprising disposing a non-impregnated tubular substrate (138) in an organic-solvent solution including the photoinitiator dissolved in the organic-solvent solution, the non-impregnated tubular substrate (138) swelling in the organic-solvent solution such that the photoinitiator diffuses into the first polymeric material, thereby impregnating the first polymeric material with the photoinitiator to provide the impregnated tubular substrate (134) in a solvent-swollen form thereof;
optionally, wherein the method further comprises disposing the solvent-swollen form of the impregnated tubular substrate (134) in water, organic solvent diffusing from the first polymeric material into the water, thereby shrinking the solvent-swollen form of the impregnated tubular substrate (134) and trapping the photoinitiator in the first polymeric material.

11. The method of any of claims 9-10, wherein the thermoplastic polyurethane includes a hard segment having one or more sulfur-based chain extenders and a soft segment having a polycarbonate moiety;
optionally, wherein the visible-light photoinitiator is camphorquinone or an analog of camphorquinone **characterized by** its absorption of electromagnetic radiation in the range of visible light from 400 nm to 650 nm.

12. The method of any of claims 9-11, wherein the impregnated tubular substrate (134) is further impregnated with a coinitiator, the coinitiator being a tertiary amine selected from ethyl-4-dimethylaminobenzoate; 4-(dimethylamino) benzonitrile; and 2-(N,N-dimethylamino)ethyl methacrylate;
optionally, wherein the coating (110) of the second polymeric material is over either an abluminal surface (116) or a luminal surface of the coated tubular substrate; or
optionally, wherein the coating (110) of the second polymeric material is over both an abluminal surface and a luminal surface of the coated tubular substrate.

13. The method of any of claims 9-12, wherein the second polymeric material is a polyacrylate salt or ester;
optionally, the method further comprises:
disposing the coated tubular substrate (132) in another aqueous solution including a therapeutic agent, thereby exchanging a proton or metal cation for a cationic therapeutic agent as a counterion to anionic carboxylate in at least a portion of functionalized sites of the second polymeric material;
optionally, wherein the therapeutic agent is an antimicrobial agent;
optionally, wherein the therapeutic agent is chlorhexidine.

14. The method of claim 13, wherein the other aqueous solution further includes a dye, thereby exchanging a proton or metal cation for a cationic dye as a counterion to anionic carboxylate in at least another portion of functionalized sites of the second polymeric material;
optionally, wherein the dye doubles as an antifungal agent;
optionally, wherein the dye is ethyl violet.

15. The method of any of claims 9-14, further comprising:
inserting a proximal end portion of the coated tubular substrate (132) into a catheter hub (104), the coated tubular substrate (132) corresponding to a catheter tube (102) of the coated catheter (100); and
inserting a distal end portion of an extension leg (106) into the catheter hub (104) for each extension leg (106) of one or more extension legs (106) of the coated catheter (100).

## Patentansprüche

1. Beschichteter Katheter (100), umfassend:
einen Katheterschlauch (102), einschließend:
ein schlauchförmiges Substrat (108) aus einem ersten polymeren Material, das für elektromagnetische Strahlung in einem Bereich von sichtbarem Licht transparent ist, wobei das erste polymere Material ein thermoplastisches Polyurethan ist, das für elektromagnetische Strahlung in dem Bereich von sichtbarem Licht von 400 nm bis 650 nm transparent ist; und
eine Beschichtung (110) aus einem zweiten polymeren Material über dem schlauchförmigen Substrat, wobei die Beschichtung durch Kettenenden des zweiten polymeren Materials, die in dem ersten polymeren Material imprägniert sind, an dem schlauchförmigen Substrat verankert ist, wobei mindestens ein Abschnitt der Kettenenden einen umgesetzten Photoinitiator für sichtbares Licht einschließt.

2. Beschichteter Katheter nach Anspruch 1, wobei das thermoplastische Polyurethan ein Hartsegment einschließt, das einen oder mehrere schwefelbasierte Kettenverlängerer aufweist; und/oder
wobei das thermoplastische Polyurethan ein Weichsegment einschließt, das einen Polycarbonat-Anteil aufweist; oder
wobei das thermoplastische Polyurethan ein Weichsegment einschließt, das einen Polyether-Anteil aufweist.

3. Beschichteter Katheter nach einem der vorstehenden Ansprüche, wobei der umgesetzte Photoinitiator für sichtbares Licht umgesetztes Campherchinon oder ein umgesetztes Analogon von Campherchinon ist.

4. Beschichteter Katheter nach einem der vorstehenden Ansprüche, wobei mindestens ein weiterer Abschnitt der Kettenenden des ersten polymeren Materials einen umgesetzten Coinitiator einschließt;
wahlweise, wobei der umgesetzte Coinitiator ein umgesetztes tertiäres Amin ist, das aus Ethyl-4-dimethylaminobenzoat; 4-(Dimethylamino)benzonitril; und 2-(N,N-Dimethylamino)ethylmethacrylat ausgewählt ist.

5. Beschichteter Katheter nach einem der vorstehenden Ansprüche, wobei die Beschichtung (110) aus dem zweiten polymeren Material entweder über einer abluminalen Oberfläche (116) oder einer luminalen Oberfläche des schlauchförmigen Substrats (108) angeordnet ist; oder
wobei die Beschichtung (110) aus dem zweiten polymeren Material sowohl über einer abluminalen Oberfläche (116) als auch einer luminalen Oberfläche des schlauchförmigen Substrats (108) angeordnet ist.

6. Beschichteter Katheter nach einem der vorstehenden Ansprüche, wobei das zweite polymere Material ein Polyacrylatsalz oder -ester ist;
wahlweise, wobei das zweite polymere Material das Polyacrylatsalz ist, wobei mindestens ein Abschnitt von funktionalisierten Stellen des zweiten polymeren Materials mit anionischem Carboxylat und einem kationischen therapeutischen Wirkstoff als Gegenion funktionalisiert ist;
wahlweise, wobei der therapeutische Wirkstoff ein antimikrobieller Wirkstoff ist;
wahlweise, wobei der therapeutische Wirkstoff Chlorhexidin ist.

7. Beschichteter Katheter nach Anspruch 6, wobei mindestens ein weiterer Abschnitt der funktionalisierten Stellen des zweiten polymeren Materials mit anionischem Carboxylat und einem kationischen Farbstoff als Gegenion funktionalisiert ist, wodurch ein sichtbarer Hinweis bereitgestellt wird, dass sich die Beschichtung (110) aus dem zweiten polymeren Material über dem schlauchförmigen Substrat (108) befindet;
wahlweise, wobei der Farbstoff zugleich als ein antimykotischer Wirkstoff dient;
wahlweise, wobei der Farbstoff Ethylviolett ist.

8. Beschichteter Katheter nach einem der vorstehenden Ansprüche, weiter umfassend:
einen Katheteransatz (104), wobei der Katheterschlauch (102) einen proximalen Endabschnitt einschließt, der in dem Katheteransatz (104) angeordnet ist; und
einen oder mehrere Verlängerungsschenkel (106), wobei jeder Verlängerungsschenkel des einen oder der mehreren Verlängerungsschenkel (106) einen distalen Endabschnitt einschließt, der in dem Katheteransatz (104) angeordnet ist.

9. Verfahren zum Herstellen eines beschichteten Katheters (100), umfassend:
Bereitstellen eines imprägnierten schlauchförmigen Substrats (134) aus einem ersten polymeren Material, das für elektromagnetische Strahlung in einem Bereich von sichtbarem Licht transparent ist, wobei das erste polymere Material ein thermoplastisches Polyurethan ist, das für elektromagnetische Strahlung in dem Bereich von sichtbarem Licht von 400 nm bis 650 nm transparent ist, wobei das erste polymere Material mit einem Photoinitiator für sichtbares Licht imprägniert ist;
Anordnen des imprägnierten schlauchförmigen Substrats (134) in einer wässrigen Lösung, die ein in der wässrigen Lösung gelöstes Monomer einschließt; und
Bestrahlen des imprägnierten schlauchförmigen Substrats (134) mit elektromagnetischer Strahlung in dem Bereich von sichtbarem Licht, für den das erste polymere Material transparent ist, wobei der Photoinitiator bei Bestrahlung des Photoinitiators eine Radikalpolymerisation des Monomers einleitet, wodurch das imprägnierte schlauchförmige Substrat (134) mit einer Beschichtung (110) aus einem zweiten polymeren Material beschichtet wird, um ein beschichtetes schlauchförmiges Substrat (132) bereitzustellen.

10. Verfahren nach Anspruch 9, weiter umfassend Anordnen eines nicht imprägnierten schlauchförmigen Substrats (138) in einer organischen Lösungsmittellösung, die den in der organischen Lösungsmittellösung gelösten Photoinitiator einschließt, wobei das nicht imprägnierte schlauchförmige Substrat (138) in der organischen Lösungsmittellösung quillt, sodass der Photoinitiator in das erste polymere Material diffundiert, wodurch das erste polymere Material mit dem Photoinitiator imprägniert wird, um das imprägnierte schlauchförmige Substrat (134) in einer lösungsmittelgequollenen Form davon bereitzustellen;
wahlweise, wobei das Verfahren weiter Anordnen der lösungsmittelgequollenen Form des imprägnierten schlauchförmigen Substrats (134) in Wasser umfasst, wobei organisches Lösungsmittel aus dem ersten polymeren Material in das Wasser diffundiert, wodurch die lösungsmittelgequollene Form des imprägnierten schlauchförmigen Substrats (134) geschrumpft wird und der Photoinitiator in dem ersten polymeren Material eingefangen wird.

11. Verfahren nach einem der Ansprüche 9-10, wobei das thermoplastische Polyurethan ein Hartsegment, das einen oder mehrere schwefelbasierte Kettenverlängerer aufweist, und ein Weichsegment, das einen Polycarbonat-Anteil aufweist, einschließt;
wahlweise, wobei der Photoinitiator für sichtbares Licht Campherchinon oder ein Analogon von Campherchinon ist, das durch seine Absorption von elektromagnetischer Strahlung in dem Bereich von sichtbarem Licht von 400 nm bis 650 nm gekennzeichnet ist.

12. Verfahren nach einem der Ansprüche 9-11, wobei das imprägnierte schlauchförmige Substrat (134) weiter mit einem Coinitiator imprägniert wird, wobei der Coinitiator ein tertiäres Amin ist, das aus Ethyl-4-dimethylaminobenzoat; 4-(Dimethylamino)benzonitril; und 2-(N,N-Dimethylamino)ethylmethacrylat ausgewählt wird;
wahlweise, wobei die Beschichtung (110) aus dem zweiten polymeren Material entweder über einer abluminalen Oberfläche (116) oder einer luminalen Oberfläche des beschichteten schlauchförmigen Substrats angeordnet ist; oder
wahlweise, wobei die Beschichtung (110) aus dem zweiten polymeren Material sowohl über einer abluminalen Oberfläche als auch einer luminalen Oberfläche des beschichteten schlauchförmigen Substrats angeordnet ist.

13. Verfahren nach einem der Ansprüche 9-12, wobei das zweite polymere Material ein Polyacrylatsalz oder -ester ist;
wahlweise, wobei das Verfahren weiter Folgendes umfasst:
Anordnen des beschichteten schlauchförmigen Substrats (132) in einer anderen wässrigen Lösung, die einen therapeutischen Wirkstoff einschließt, wodurch ein Proton oder ein Metallkation gegen einen kationischen therapeutischen Wirkstoff als Gegenion zu anionischem Carboxylat in mindestens einem Abschnitt von funktionalisierten Stellen des zweiten polymeren Materials ausgetauscht wird;
wahlweise, wobei der therapeutische Wirkstoff ein antimikrobieller Wirkstoff ist;
wahlweise, wobei der therapeutische Wirkstoff Chlorhexidin ist.

14. Verfahren nach Anspruch 13, wobei die andere wässrige Lösung weiter einen Farbstoff einschließt, wodurch ein Proton oder ein Metallkation gegen einen kationischen Farbstoff als Gegenion zu anionischem Carboxylat in mindestens einem weiteren Abschnitt von funktionalisierten Stellen des zweiten polymeren Materials ausgetauscht wird;
wahlweise, wobei der Farbstoff zugleich als ein antimykotischer Wirkstoff dient;
wahlweise, wobei der Farbstoff Ethylviolett ist.

15. Verfahren nach einem der Ansprüche 9-14, weiter umfassend:
Einsetzen eines proximalen Endabschnitts des beschichteten schlauchförmigen Substrats (132) in einen Katheteransatz (104), wobei das beschichtete schlauchförmige Substrat (132) einem Katheterschlauch (102) des beschichteten Katheters (100) entspricht; und
Einsetzen eines distalen Endabschnitts eines Verlängerungsschenkels (106) in den Katheteransatz (104) für jeden Verlängerungsschenkel (106) von einem oder mehreren Verlängerungsschenkeln (106) des beschichteten Katheters (100).

## Revendications

1. Cathéter revêtu (100), comprenant :
un tube (102) de cathéter incluant :
un substrat tubulaire (108) d'un premier matériau polymère transparent au rayonnement électromagnétique dans une plage de lumière visible, dans lequel le premier matériau polymère est un polyuréthane thermoplastique transparent au rayonnement électromagnétique dans la plage de la lumière visible allant de 400 nm à 650 nm ; et
un revêtement (110) d'un deuxième matériau polymère sur le substrat tubulaire, le revêtement étant ancré au substrat tubulaire par des extrémités de chaîne du deuxième matériau polymère imprégné dans le premier matériau polymère, au moins une partie des extrémités de chaîne incluant un photo-initiateur à lumière visible consommé.

2. Cathéter revêtu selon la revendication 1, dans lequel le polyuréthane thermoplastique inclut un segment dur présentant un ou plusieurs agents d'allongement de chaîne à base de soufre ; et/ou
dans lequel le polyuréthane thermoplastique inclut un segment souple présentant un motif polycarbonate ; ou
dans lequel le polyuréthane thermoplastique inclut un segment souple présentant un motif polyéther.

3. Cathéter revêtu selon l'une quelconque des revendications précédentes, dans lequel le photo-initiateur à lumière visible consommé est de la camphorquinone consommée ou un analogue de la camphorquinone consommé.

4. Cathéter revêtu selon l'une quelconque des revendications précédentes, dans lequel au moins une autre partie des extrémités de chaîne du premier matériau polymère inclut un co-initiateur consommé ;
éventuellement, dans lequel le co-initiateur consommé est une amine tertiaire consommée choisie parmi l'éthyl-4-diméthylaminobenzoate ; le 4-(diméthylamino)benzonitrile ; et le méthacrylate de 2-(N,N-diméthylamino)éthyle.

5. Cathéter revêtu selon l'une quelconque des revendications précédentes, dans lequel le revêtement (110) du deuxième matériau polymère est sur soit une surface abluminale (116) soit une surface luminale du substrat tubulaire (108) ; ou
dans lequel le revêtement (110) du deuxième matériau polymère est sur à la fois une surface abluminale (116) et une surface luminale du substrat tubulaire (108).

6. Cathéter revêtu selon l'une quelconque des revendications précédentes, dans lequel le deuxième matériau polymère est un sel ou ester de polyacrylate ;
éventuellement, dans lequel le deuxième matériau polymère est le sel de polyacrylate, au moins une partie de sites fonctionnalisés du deuxième matériau polymère étant fonctionnalisée avec du carboxylate anionique et un agent thérapeutique cationique en tant que contre-ion ;
éventuellement, dans lequel l'agent thérapeutique est un agent antimicrobien ;
éventuellement, dans lequel l'agent thérapeutique est la chlorhexidine.

7. Cathéter revêtu selon la revendication 6, dans lequel au moins une autre partie des sites fonctionnalisés du deuxième matériau polymère est fonctionnalisée avec du carboxylate anionique et un colorant cationique en tant que contre-ion, de sorte à fournir une indication visible que le revêtement (110) du deuxième matériau polymère est sur le substrat tubulaire (108) ;
éventuellement, dans lequel le colorant fait également fonction d'agent antifongique ;
éventuellement, dans lequel le colorant est le violet d'éthyle.

8. Cathéter revêtu selon l'une quelconque des revendications précédentes, comprenant en outre :
une embase (104) de cathéter, le tube (102) de cathéter incluant une partie d'extrémité proximale disposée dans l'embase (104) de cathéter ; et
une ou plusieurs pattes d'extension (106), chaque patte d'extension des une ou plusieurs pattes d'extension (106) incluant une partie d'extrémité distale disposée dans l'embase (104) de cathéter.

9. Procédé de fabrication d'un cathéter revêtu (100), comprenant :
l'obtention d'un substrat tubulaire imprégné (134) d'un premier matériau polymère transparent au rayonnement électromagnétique dans une plage de lumière visible, dans lequel le premier matériau polymère est un polyuréthane thermoplastique transparent au rayonnement électromagnétique dans la plage de la lumière visible allant de 400 nm à 650 nm, le premier matériau polymère étant imprégné d'un photo-initiateur à lumière visible ;
le fait de disposer le substrat tubulaire imprégné (134) dans une solution aqueuse incluant un monomère dissous dans la solution aqueuse ; et
l'irradiation du substrat tubulaire imprégné (134) avec un rayonnement électromagnétique dans la plage de la lumière visible à laquelle le premier matériau polymère est transparent, le photo-initiateur initiant une polymérisation radicalaire du monomère lors de l'irradiation du photo-initiateur, de sorte à revêtir le substrat tubulaire imprégné (134) d'un revêtement (110) d'un deuxième matériau polymère pour fournir un substrat tubulaire revêtu (132).

10. Procédé selon la revendication 9, comprenant en outre le fait de disposer un substrat tubulaire non imprégné (138) dans une solution de solvant organique incluant le photo-initiateur dissous dans la solution de solvant organique, le substrat tubulaire non imprégné (138) gonflant dans la solution de solvant organique de telle sorte que le photo-initiateur se diffuse dans le premier matériau polymère, de sorte à imprégner le premier matériau polymère avec le photo-initiateur pour fournir le substrat tubulaire imprégné (134) sous une forme gonflée par le solvant de celui-ci ;
éventuellement, dans lequel le procédé comprend en outre le fait de disposer la forme gonflée par le solvant du substrat tubulaire imprégné (134) dans de l'eau, le solvant organique se diffusant à partir du premier matériau polymère dans l'eau, de sorte à rétrécir la forme gonflée par le solvant du substrat tubulaire imprégné (134) et piégeant le photo-initiateur dans le premier matériau polymère.

11. Procédé selon l'une quelconque des revendications 9 à 10, dans lequel le polyuréthane thermoplastique inclut un segment dur présentant un ou plusieurs agents d'allongement de chaîne à base de soufre et un segment souple présentant un motif polycarbonate ;
éventuellement, dans lequel le photo-initiateur à lumière visible est la camphorquinone ou un analogue de la camphorquinone **caractérisé par** son absorption de rayonnement électromagnétique dans la plage de la lumière visible de 400 nm à 650 nm.

12. Procédé selon l'une quelconque des revendications 9 à 11, dans lequel le substrat tubulaire imprégné (134) est en outre imprégné avec un co-initiateur, le co-initiateur étant une amine tertiaire choisie parmi l'éthyl-4-diméthylaminobenzoate ; le 4-(diméthylamino)benzonitrile ; et le méthacrylate de 2-(N,N-diméthylamino)éthyle ;
éventuellement, dans lequel le revêtement (110) du deuxième matériau polymère est sur soit une surface abluminale (116), soit une surface luminale du substrat tubulaire revêtu ; ou
éventuellement, dans lequel le revêtement (110) du deuxième matériau polymère est sur à la fois une surface abluminale et une surface luminale du substrat tubulaire revêtu.

13. Procédé selon l'une quelconque des revendications 9 à 12, dans lequel le deuxième matériau polymère est un sel ou ester de polyacrylate ;
éventuellement, le procédé comprend en outre :
le fait de disposer le substrat tubulaire revêtu (132) dans une autre solution aqueuse incluant un agent thérapeutique, de sorte à échanger un proton ou un cation métallique contre un agent thérapeutique cationique en tant que contre-ion du carboxylate anionique dans au moins une partie de sites fonctionnalisés du deuxième matériau polymère ;
éventuellement, dans lequel l'agent thérapeutique est un agent antimicrobien ;
éventuellement, dans lequel l'agent thérapeutique est la chlorhexidine.

14. Procédé selon la revendication 13, dans lequel l'autre solution aqueuse inclut en outre un colorant, de sorte à échanger un proton ou un cation métallique contre un colorant cationique en tant que contre-ion du carboxylate anionique dans au moins une autre partie de sites fonctionnalisés du deuxième matériau polymère ;
éventuellement, dans lequel le colorant fait également fonction d'agent antifongique ;
éventuellement, dans lequel le colorant est le violet d'éthyle.

15. Procédé selon l'une quelconque des revendications 9 à 14, comprenant en outre :
l'insertion d'une partie d'extrémité proximale du substrat tubulaire revêtu (132) dans une embase (104) de cathéter, le substrat tubulaire revêtu (132) correspondant à un tube (102) de cathéter du cathéter revêtu (100) ; et
l'insertion d'une partie d'extrémité distale d'une patte d'extension (106) dans l'embase (104) de cathéter pour chaque patte d'extension (106) des une ou plusieurs pattes d'extension (106) du cathéter revêtu (100).
